# EUROPÄISCHE PATENTSCHRIFT

(11) **EP 0 384 476 B1**
(45) Veröffentlichungstag und Bekanntmachung des Hinweises auf die Patenterteilung: **06.10.1993**
(21) Anmeldenummer: 90103502.2
(22) Anmeldetag: 23.02.1990
(51) Int. Cl.: A61M 25/00

(54) **Einmalkatheter zur Harnableitung**
Disposable catheter for urine diversion
Cathéter à jeter pour évacuation d'urine

(30) Priorität: 23.02.1989 DE 3905552
(43) Veröffentlichungstag der Anmeldung: 29.08.1990
(73) Patentinhaber: LIPAC, LIEBENZELLER VERPACKUNGSGESELLSCHAFT MBH, D-75378 Bad Liebenzell (DE)
(72) Erfinder: Hutzler, Walter, 7263 Bad Liebenzell (DE)
(74) Vertreter: KOHLER SCHMID + PARTNER

(56) Entgegenhaltungen:
- DE-A- 2 727 273
- DE-A- 3 441 586
- DE-C- 330 284
- FR-B- 2 247 262
- US-A- 3 665 928
- US-A- 4 248 234

## Beschreibung

Die Erfindung geht aus von einem Einmalkatheter zur Harnableitung. Derartige Katheter sind unter der Fachbezeichnung "Tiemann-Katheter" bekanntgeworden.

Zum Katheterismus der Harnblase werden Gummi- oder Kunststoffkatheter verwendet. Sie dienen in erster Linie der Harnableitung. Die röhrenförmigen Instrumente sind einläufig und weisen in Spitzennähe Augen für die Harnableitung und endständig einen Trichter als Verbindungselement an abführende Geräte auf. Benötigt werden die zur einmaligen Verwendung vorgesehenen Katheter von Personen, bei denen die Harnblasenmuskulatur vollständig oder teilweise gelähmt ist. Jeder Katheterismus muß unter strenger Beachtung der Regeln der Asepsis durchgeführt werden, hat schonend und vorsichtig zu erfolgen und wird vom Arzt oder Pflegepersonal, oder von dem Patienten durch Selbstkatheterisierung durchgeführt.

Der bekannte Einmalgebrauchs-Blasenkatheter nach Tiemann weist eine gekrümmt, konisch zulaufende und am Ende kugelförmig verdickte Spitze auf, die den anatomischen Gegebenheiten des Harnröhrenverlaufs beim Mann angepaßt ist. Die kugelförmige Verdickung ist unmittelbar an eine nicht sehr stark ausgeprägte Verjüngung angeformt. Diese Maßnahme ist erforderlich, damit die gekrümmte Spitze eine gewisse Eigensteifigkeit erhält und die kugelförmige Anformung bei axialer Belastung nicht entgegen der Einführrichtung des Katheters abgebogen wird. Ferner hat der Katheterschaft des bekannten Blasenkatheters eine glatte Oberfläche.

Bei dem bekannten Katheter nach Tiemann muß beim Einführen des Katheters genau darauf geachtet werden, daß die Katheterspitze entsprechend dem Harnröhrenverlauf ausgerichtet ist. Dies ist, bedingt durch die Behinderung des Patienten, häufig ein schwer zu bewältigendes Problem.

Weiterhin wirkt die Aussteifung des Spitzenendes, die aufgrund der gekrümmten Form dieser Spitze notwendig ist, einem schonenden Katheterismus entgegen.

Die glatte Oberfläche des Katheterschafts bewirkt, daß sich das Instrument an der Harnröhrenwand mehr oder weniger festsaugt und Gleitmittel können diesem Ansaugeffekt nur bedingt entgegenwirken, weil das Gleitmittel beim Einführen des Katheters in die Harnröhre entgegen der Bewegungsrichtung des Katheters zurückgeschoben wird.

Weiterhin sind noch Einmalkatheter benannt nach Nélaton und Mercier bekanntgeworden. Diese Katheter weisen als Spitze einen kugelförmigen Abschluß auf. Diese haben den Nachteil, daß beim Einführen des Katheters durch die Kalotte ein erhöhter Druck auf die Urethralschleimhaut ausgeübt wird und die kurze runde Spitze öffnet die Mm. sphinkter vesicae relativ schnell. Eine derartige Spitzenform erschwert einen schonenden, vorsichtigen und sicheren Katheterismus.

Schließlich weisen die bekannten Einmalkatheter Augen in Spitzennähe auf, deren Randzonen zur freien glatten Oberfläche des Katheterschaftes hin beim Katheterismus die Schleimhaut der Urethra verletzen können. Da die Augen normalerweise mit Stanzmessern aus dem Katheterschaft herausgeschnitten werden, weisen die Augen an den Schnittstellen scharfe Kanten auf. Diese Kanten scheren beim Einführen des Katheters Schleimhautpartikeln ab. Ebenfalls können die Augen beim Durchdringen der Mm. sphinkter vesicae Verletzungen hervorrufen.

Aus der US-A-4,248,234 ist ein Katheter bekannt, der sich aus einem ersten flexiblen, im Spitzenbereich konisch verlaufenden Schlauch und einem zweiten Schlauch zusammensetzt. Der erste Schlauch ist sowohl am proximalen Ende wie auch am distalen Ende (hier über einen Trichter) geöffnet und der zweite Schlauch ist in den ersten Schlauch einführbar. Ist der zweite Schlauch, der an seinem proximalen Ende geschlossen ist, in den ersten Schlauch eingeführt, so kann der zweite Schlauch druckbeaufschlagt werden, damit der erste Schlauch sich mehr oder weniger stark versteift. Bei diesem Versteifungsvorgang tritt das geschlossene Ende des zweiten Schlauchs aus der proximalen Öffnung des ersten Schlauchs heraus und verschließt diese Öffnung, indem sich das geschlossene Ende des zweiten Schlauchs kugelförmig verdickend, eine Einschnürung bildend auswölbt. Ist der zweite Schlauch druckbeaufschlagt, so ist der erste Schlauch richtungsstabil versteift.

Der Erfindung liegt die Aufgabe zugrunde, einen Einmalkatheter der eingangs genannten Art dahingehend weiterzubilden, daß der Katheterismus einfacher, schonender und sicherer erfolgen kann.

Diese Aufgabe wird durch die Merkmale des Anspruchs 1 gelöst.

Der erfindungsgemäße Katheter hat damit den wesentlichen Vorteil, daß aufgrund der Spitzenform die Mm. sphinkter vesicae schonender geöffnet werden und daß die Weitung der Harnröhre entsprechend der Länge und des Neigungswinkels der sich konisch erweiternden Mantelfläche der Spitze in Richtung Katheterschaft sukzessiv erfolgt. Die kugelförmige Verdickung kann kleiner gehalten werden als bei einer gekrümmten Spitze und sie kann auch elastischer an der Verjüngung der Spitze angeformt sein, ohne daß bei einer axialen Belastung der erfindungsgemäßen Spitze die Richtungsstabilität des erfindungsgemäBen Katheters einschränkt werden würde. Über den kurzen halsförmigen Abschnitt, der sich zwischen der konischen Verjüngung und der kugelförmigen Verdickung erstreckt, wird die kugelförmige Verdickung in hohem Maße elastisch und kann sich Richtungsänderungen der Harnröhre anpassen. Die konstruktive Ausgestaltung der Spitze gewährleistet gleichermaßen einen schonenden wie auch sicheren und einfachen Katheterismus. Die Spitze ist mit dem Katheterschaft formschlüssig und fest verbunden. Dies hat den Vorteil, daß je nach Verwendungszweck des erfindungsgemäßen Katheters unterschiedliche Spitzen in Bezug auf ihre Flexibilität, Größe und Länge mit dem Katheterschaft verbunden werden können.

Bei einer Weiterbildung der Erfindung sind die Augen im Randbereich nach außen und innen abgerundet.

Dies hat den Vorteil, daß beim Einführen des erfindungsgemäßen Katheters sowohl die Urethralschleimhaut wie auch die Mm. sphinkter vesicae besonders schonend belastet werden. Beim Katheterismus werden Abscherungen von Schleimhautpartikeln weitgehendst vermieden.

Weiterhin weist die Oberfläche des Katheterschaftes mikroskopisch kleine dicht aneinandergereihte halbabgerundete Höcker, vorzugsweise Kalotten, auf.

Dies hat den Vorteil, daß sich die Urethralschleimhaut und die Manteloberfläche des Katheterschaftes nicht aneinander festsaugen können. Reizungen dieser Art, die der Patient als unangenehm und schmerzhaft empfindet, können wesentlicht verringert werden. Die Berührungen der Harnröhreninnenwand und der Manteloberfläche des Katheterschaftes sind punktueller Art. Es entstehen keine großflächigen Adhärenzen.

In weiterer Ausgestaltung der Erfindung ist in Täler zwischen den Höckern Gleitmittel einlagerbar.

Dies hat den Vorteil, daß beim Einführen des erfindungsgemäßen Katheters in die Harnröhre das Gleitmittel nicht flächenhaft sofort zurückgeschoben werden kann. Aus den Tälern wird das Gleitmittel nach und nach abgegeben, so daß sich die Vorzüge des Gleitmittels über die gesamte Länge der Harnröhre auswirken.

In weiterer Ausbildung der Erfindung sind die halbabgerundeten Höcker auch an der Spitze ausgebildet. Sie verbessern die Gleitwerte der Spitze. Diese Maßnahme trägt zu einer weiteren Schonung der Urethralschleimhaut beim Einführen des erfindungsgemäßen Katheters bei.

Es versteht sich, daß die vorstehend genannten und die nachstehend noch erläuterten Merkmale nicht nur in der jeweils angegebenen Kombination, sondern auch in anderen Kombinationen oder in Alleinstellung einsetzbar sind, ohne den Rahmen der vorliegenden Erfindung zu verlassen.

Weitere Vorteile ergeben sich aus der Beschreibung und der beigefügten Zeichnung.

Ausführungsbeispiele der Erfindung sind in der Zeichnung dargestellt und werden in der nachfolgenden Beschreibung näher erläutert. Es zeigt:
- Fig. 1: eine perspektivische Ansicht eines erfindungsgemäßen Einmalkatheters zur Harnableitung;
- Fig. 2: eine Teilansicht der Spitze des erfindungsgemäßen Einmalkatheters in vergrößertem Maßstab;
- Fig. 3: einen Abschnitt eines Katheterschaftes im Bereich von Augen im vergrößerten Maßstab;
- Fig. 4: einen Schnitt IV-IV gemäß Fig. 3;
- Fig. 5: einen Querschnitt des Katheterschaftes eines erfindungsgemäßen Einmalkatheters in vergrößertem Maßstab;
- Fig. 6: einen Abschnitt einer Seitenansicht gemäß Fig. 5;
- Fig. 7: eine weitere Teilansicht eines erfindungsgemäßen Einmalkatheters.

Die einzelnen Figuren der Zeichnung zeigen den erfindungsgemäßen Gegenstand teilweise nicht maßstäblich. Die Gegenstände der einzelnen Figuren sind teilweise stark vergrößert, schematisiert und vereinfacht dargestellt, damit ihr Aufbau besser gezeigt werden kann.

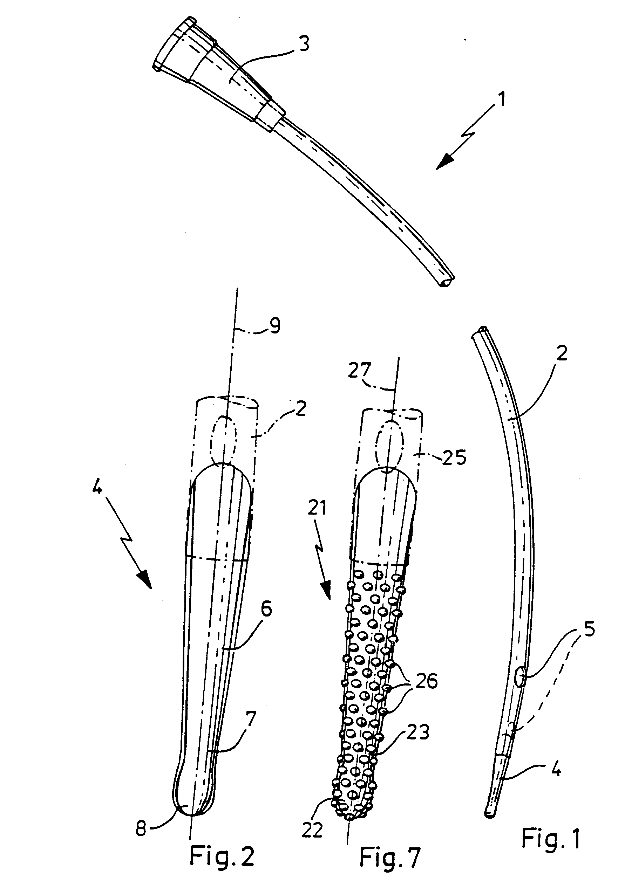

In Fig. 1 ist mit 1 ein Einmalkatheter zur Harnableitung dargestellt. Der Einmalkatheter zur Harnableitung 1 setzt sich aus einem Katheterschaft 2, einem Trichter 3 und aus einer Spitze 4 zusammen. Der Katheterschaft 2 weist in Spitzennähe Augen 5 auf. Die Spitze 4 ist, als getrenntes Teil gefertigt, mit dem Katheterschaft 2 fest und formschlüssig verbunden. Die Augen 5 können an unterschiedlichen Stellen am Umfang des Katheterschaftes angeordnet sein. Der Katheter selbst ist aus körperverträglichen Kunststoffmaterialien gefertigt. Die Spitze 4 wie auch der Katheterschaft 2 sind in einem hohen Maße flexibel, sie können aber dennoch richtungsstabil geführt werden.

Fig. 2 zeigt in vergrößertem Maßstab, wie die Spitze 4 in den Katheterschaft 2 eingefügt ist. Ein Teilstück des Katheterschaftes 2 ist in gestrichelten Linien gezeigt. Die Spitze 4 weist in Verlängerung des Katheterschaftes 2 eine konische Verjüngung auf, die in einen halsförmigen Abschnitt 7 übergeht, an den sich eine kugelförmige Verdickung 8 anschließt. Die Spitze 4 endet in der kugelförmigen Verdickung 8. Über den halsförmigen Abschnitt 7 ist die kugelförmige Verdickung 8 flexibel an der Verjüngung 6 angeformt. Die Spitze 4 kann sich somit Richtungsänderungen des Harnröhrenverlaufs sicher und schonend anpassen, ohne daß sie die dafür notwendige Richtungsstabilität verliert. Die Achse 9 ist die Achse des Katheterschaftes 2 und die Achse der Spitze 4.

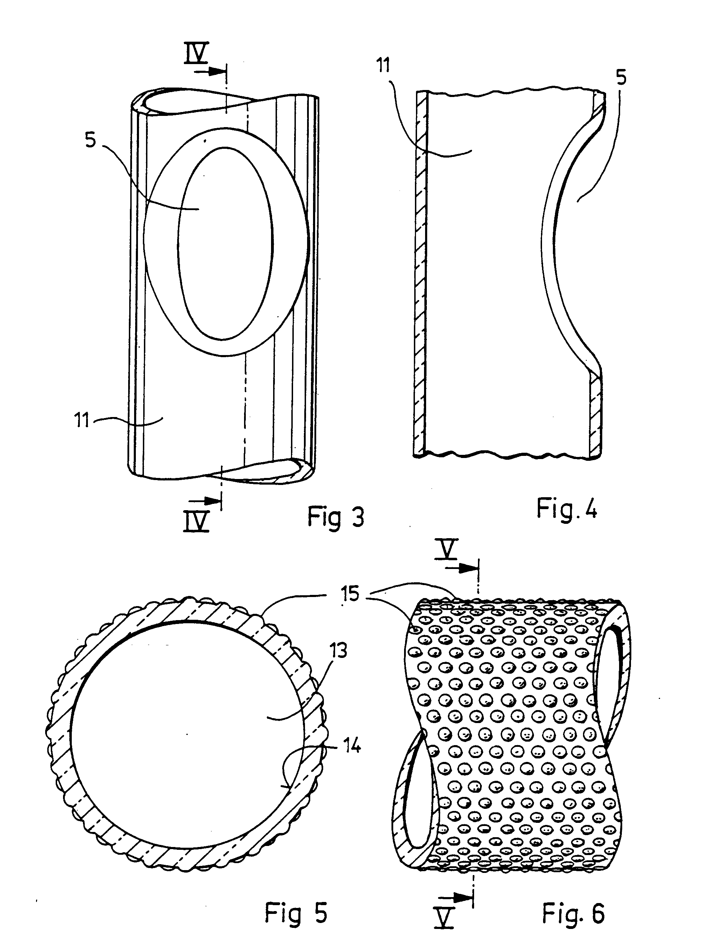

Fig. 3 zeigt in vergrößertem Maßstab einen Abschnitt des Katheterschafts 2, der an einem Schaftabschnitt 11 das Auge 5 aufweist. Das Auge 5 ist in den Schaftabschnitt 11 so eingeformt, daß keine Kanten entstehen und Randzonen des Auges 5 fließend in die Schaftabschnittsoberfläche übergehen.

Fig. 4 zeigt das Auge 5 im Schnitt IV-IV der Fig. 3. Der Schnitt verdeutlicht, daß der Schaftabschnitt 11, der das Auge 5 aufnimmt, keine Kanten aufweist, die Verletzungen der Urethralschleimhaut hervorrufen könnten.

Fig. 5 zeigt einen Querschnitt eines Katheterschaftes 2 in vergrößertem Maßstab im Schnitt V-V einer Fig. 6. Ein großes Lumen 13 bei kleinem Schaftdurchmesser gewährleistet die schnelle Abführung der Harnflüssigkeit. Das Lumen 13 ist durch eine Schaftinnenwand 14 begrenzt. Auf der Manteloberfläche des Katheterschaftes 2 sind Höcker 15 angeordnet. Um die Höcker 15 sichtbar zu machen, sind sie gegenüber dem Lumen 13 überproportional vergrößert dargestellt. Über die Höcker 15 wird ein schonendes Gleiten des Katheterschaftes 2 in der Harnröhre erreicht. Der Katheterschaft 2 kann sich nicht an der Urethralschleimhaut der Harnröhre festsaugen. Die Höcker 15 können halbabgerundete Erhebungen, vorzugsweise Kalotten, sein.

Fig. 6 zeigt eine Seitenansicht der Fig. 5. Die Höcker 15 sind auf der Mantelfläche des Katheterschaftes 2 gleichmäßig oder ungleichmäßig eng aneinandergereiht verteilt. Es entsteht eine perlige Oberflächenstruktur. In den Tälern der Höcker 15 kann sich Gleitmittel ansammeln, das nach und nach beim Einführen des erfindungsgemäßen Katheters abgegeben wird.

Mit dem erfindungsgemäßen Einmalkatheter zur Harnableitung 1 wird mittels seiner Spitze 4 erreicht, daß sich der Katheter jeder Biegung der Harnröhre schonend anpaßt. Er kann unproblematisch eingeführt werden, d.h. auch für den behinderten Patienten ergeben sich hierbei keine Probleme. Die Augen 5 des neuen Katheters sind atraumatisch ausgeformt. Damit wird eine optimale Schonung der Urethralschleimhaut sowie der Mm. sphinkter vesicae erzielt. Die Oberfläche des Katheterschaftes 2 des erfindungsgemäßen Katheters hat eine perlige Struktur, die in Verbindung mit einem Gleitmittel optimale Gleiteigenschaften bietet.

Fig. 7 zeigt eine Spitze 21 mit einer kugelförmigen Verdickung 22, die das Ende eines halsförmigen Abschnitts 23 bildet. Der halsförmige Abschnitt 23 weist zur kugelförmigen Verdickung 22 hin eine Verjüngung auf. Die Spitze 21 ist mit dem der kugelförmigen Verdickung 22 gegenüberliegenden Ende mit einem Katheterschaft 25 fest verbunden. Auf der Oberfläche der Spitze 21 sind, auch im Bereich der kugelförmigen Verdickung 22, halbabgerundete Höcker 26 flächendeckend ausgebildet. Bei den halbabgerundeten Höckern 26 handelt es sich bevorzugt um Kalotten. Die Spitze 21 verläuft zum Katheterschaft 25 koaxial. Sie haben als gemeinsame Achse eine Achse 27.

## Patentansprüche

1. Einmalkatheter zur Harnableitung mit einem flexiblen Katheterschaft (2), der Augen (5) aufweist und am distalen Ende von einem Trichter (3) und am proximalen Ende von einer flexiblen Spitze (4), die mit dem Katheterschaft (2) formschlüssig und fest verbunden ist, begrenzt ist, wobei sich die Spitze (4) geradeverlaufend konisch verjüngt, derart, daß bei Einführung des Katheters in die Harnröhre die Weitung derselben sukzessiv erfolgt, und die Verjüngung der Spitze (4) in einen kurzen halsförmigen Abschnitt (7) übergeht, der sich zwischen der konischen Verjüngung u. einer kugelförmigen, flexiblen Verdickung (8) erstreckt, die an den halsförmigen Abschnitt (7) angeformt ist.

2. Einmalkatheter nach Anspruch 1, dadurch gekennzeichnet, daß die Augen (5) im Randbereich nach außen und innen abgerundet sind.

3. Einmalkatheter nach einem der Ansprüche 1 oder 2, dadurch gekennzeichnet, daß die Oberfläche des Katheterschaftes (2) mikroskopisch kleine dicht aneinandergereihte halbabgerundete Höcker (15), bevorzugt Kalotten, aufweist.

4. Einmalkatheter nach Anspruch 3, dadurch gekennzeichnet, daß in Tälern zwischen den Höckern (15) Gleitmittel einlagerbar ist.

5. Einmalkatheter nach Anspruch 3, dadurch gekennzeichnet, daß die halbabgerundeten Höcker (26) an der Oberfläche der Spitze (21) des Katheterschaftes (25) ausgeformt sind.

## Claims

1. Disposable catheter for urinary drainage with a flexible catheter shank (2), exhibiting window openings (5) and bordered at the distal end by a funnel (3) and at the proximal end by a flexible tip (4) which is form-fit and firmly attached to the catheter shank (2), whereby the tip (4) is straight and conically tapered in such a fashion, that, when introducing the catheter into the urethra, the widening of the urethra transpires successively and the tapering of the tip (4) exhibits a transition to a short neck-shaped section (7) extending between the conical taper and a ball-shaped flexible enlargement (8) is formed onto the neck-shaped section (7).

2. Disposable catheter according to claim 1, characterized in that the window openings (5) are rounded towards the outside and the inside in the bordering regions.

3. Disposable catheter according to one of the claims 1 or 2, characterized in that the surface of the catheter shank (2) exhibits small densely arranged rounded protrusions (15), preferentially spherical caps.

4. Disposable catheter according to claim 3, characterized in that lubricant can be stored in the valleys between the protrusions (15).

5. Disposable catheter according to claim 3, characterized in that the rounded protrusions (26) are formed on the surface of the tip (21) of the catheter shank (25).

## Revendications

1. Cathéter à utiliser une fois, pour l'évacuation d'urine, comportant un tronc de cathéter flexible (2) qui présente des oeillets (5) et qui est délimité à l'extrémité distale par un entonnoir (3) et à l'extrémité proximale par une pointe flexible (4) raccordée rigidement et par une liaison par la forme au tronc de cathéter (2), la pointe (4) diminuant de façon conique dans un tracé rectiligne de façon que, lors de l'introduction du cathéter dans l'urètre, l'élargissement de celui-ci ait lieu de façon successive, le rétrécissement de la pointe (4) se transformant en une courte section en forme de col (7) qui s'étend entre le rétrécissement conique et un épaississement flexible (8) en forme de sphère qui est façonné sur la section (7) en forme de col.

2. Cathéter à utiliser une fois, suivant la revendication 1, caractérisé en ce que les oeillets (5) sont arrondis vers l'extérieur et vers l'intérieur dans la zone du bord.

3. Cathéter à utiliser une fois, suivant l'une ou l'autre des revendications 1 et 2, caractérisé en ce que la surface du tronc de cathéter (2) présente des bosses (15) microscopiquement petites mises en files serrées et à moitié arrondies, de préférence des calottes.

4. Cathéter à utiliser une fois, suivant la revendication 3, caractérisé en ce qu'un agent lubrifiant peut être stocké dans des sillons entre les bosses (15).

5. Cathéter à utiliser une fois, suivant la revendication 3, caractérisé en ce que les bosses (26) à moitié arrondies sont façonnées sur la surface de la pointe (21) du tronc de cathéter (25).
